# EUROPÄISCHE PATENTSCHRIFT

(11) **EP 3 457 047 B1**
(45) Veröffentlichungstag und Bekanntmachung des Hinweises auf die Patenterteilung: **04.01.2023**
(21) Anmeldenummer: 17191238.9
(22) Anmeldetag: 15.09.2017
(51) Int. Cl.: F24F 8/10, F24F 13/20, F24F 13/28

(54) **PERSONALISIERTE LUFTREINIGUNGSVORRICHTUNG**
PERSONALISED AIR PURIFICATION DEVICE
DISPOSITIF DE PURIFICATION D'AIR PERSONNALISÉ

(43) Veröffentlichungstag der Anmeldung: 20.03.2019
(73) Patentinhaber: IQAir AG, 9403 Goldach (CH)
(72) Erfinder: Hammes, Frank, 9403 Horn (CH); Susana, Christian, 6833 Fraxern (AT)
(74) Vertreter: Riebling, Peter

(56) Entgegenhaltungen:
- WO-A1-2012/023655
- DE-U1-202009 001 190
- US-A1- 2007 018 415
- US-A1- 2011 030 560

## Beschreibung

Die Erfindung betrifft eine Luftreinigungsvorrichtung zur personalisierten Luftzufuhr nach dem Oberbegriff des Patentanspruches 1.

Unter dem Begriff "personalisiert" wird im Sinne der Erfindung verstanden, dass die gereinigte oder in anderer Weise aufbereitete Raumluft direkt und individuell einem Benutzer zugeführt werden kann.

Besonders sensitive Personen, die viel Zeit an einem Platz in Großraumbüros und in anderen Räumen verbringen, haben vermehrt das Bedürfnis, Luft zu atmen, die gereinigt ist und/oder beduftet sein kann.

Es besteht daher ein Bedürfnis, die Luft in Räumen noch zusätzlich zu reinigen und/oder zu beduften und jeweils einem Benutzer personalisiert zuzuführen. Dies auch deshalb, weil Klimaanlagen keine völlige Allergiefreiheit (Freiheit von Blüten- und Gräserpollen) und Geruchsfreiheit der zugeführten Raumluft erreichen können. Des Weiteren deshalb, weil Raumluftreiniger, sofern vorhanden, nicht die gesamte Raumluft in einem Raum, aufgrund ständig aktiver Luftverschmutzungsquellen, vollständig reinigen können.

Mit dem Gegenstand der US 9,375,547 B2 und US 9,144,697 B2 gab es Versuche, gereinigte oder in anderer Weise aufbereitete Raumluft individualisiert einem Benutzer zuzuführen. Nachteil der dort beschriebenen Anordnung ist, dass die Luftzuführung in der Art einer Frisierhaube stattfindet, d. h. der Benutzer muss sich unterhalb einer Lufthutze befinden, die einen gegen den Kopf des Benutzers gerichteten Luftausblasstrahl erzeugt.

Aus der US 8,414,671 B2 ist es ferner bekannt, den Kopf des Benutzers in einem zentralen kastenförmigen Gerät zu lagern, um gezielt dem Benutzer einen auf ihn abgestimmten gereinigten Luftstrom zuzuführen.

Nachteil der genannten Druckschriften ist die Notwendigkeit der Anordnung von eigenen Luftzuführvorrichtungen, die den Benutzer möglicherweise in seiner Bewegungsfreiheit einschränken und weiterer Nachteil ist die schwierige Handhabung, denn die gereinigte Luft wird von einem auf den Boden aufgestellten Gerät erzeugt, welches über einen relativ langen Schlauch mit der Luftzuführungshutze verbunden ist.

Weiterer Nachteil dieser bekannten Technik ist, dass sich im Gerät selbst und in den langen Luftzuführungsschläuchen Schmutzteile und Bakterien absetzen können, die zu einer Verkeimung der zugeführten Luft führen.

Aus der US 2007/018415 A1 und US 2011/030560 A1 sind Luftreinigungsvorrichtungen gemäß den Merkmalen des Oberbegriffs des Anspruchs 1 bekannt.

Der Erfindung liegt deshalb die Aufgabe zugrunde, eine Luftreinigungsvorrichtung der eingangs genannten Art so weiterzubilden, dass die Möglichkeit einer personalisierten Luftzufuhr, eine leichte Handhabbarkeit des Gerätes und eine verbesserte Betriebssicherheit gewährleistet sind.

Zur Lösung der gestellten Aufgabe ist die Erfindung durch die technische Lehre des Anspruchs 1 gekennzeichnet.

Bevorzugte Ausführungsformen der Erfindung sind in den abhängigen Ansprüchen definiert.

Wo immer eine Person für längere Zeit an einem definierten Platz, wie z.B. Arbeitstisch oder im Bett Zeit verbringt und die Luft nicht frei von Luftfremdstoffen ist, ist eine personalisierte Luftreinigungsvorrichtung von Vorteil.

Gegenüber Raumluftreinigern, die versuchen die ganze Luft in einem Innenraum zu reinigen und oft nur zu einer kleinen Verbesserung der Raumluft beitragen, da sie nicht genug Raumluft reinigen, um mit den ständigen Luftverschmutzungsquellen mitzuhalten, kann eine personalisierte Luftreinigungsvorrichtung wirksamer, stromsparender und leiser sein, da sie nur so viel Luft reinigt wie notwendig ist, um die unmittelbare Atemzone des Nutzers zu reinigen.

Dadurch, dass das Gerät sehr nahe an und der Auslass im optimalen Winkel der Atemzone des Nutzer positioniert werden kann, vermischt sich die saubere Luft vom personalisierten Luftreiniger nicht mit Schadstoffen aus Schadstoffquellen im Raum. Der Nutzer kann extrem sauberer Luft atmen.

Ein bevorzugtes Merkmal der Erfindung ist , dass die personalisierte Luftreinigungsvorrichtung in der Ausführung als Tischgerät ein Gehäuse aufweist, das in den Abmessungen und der Formgebung etwa einer - für Sportwettbewerbe verwendeten - Diskusscheibe entspricht, wie sie als Wurfgerät im Sport bekannt ist. Kennzeichnend für diese Form ist, dass die Luftaufbereitung und Luftabgabe in einem relativ kleinen, rotationssymmetrischen Gehäuse stattfindet, so dass sich erstmals die Möglichkeit bietet, eine solche Luftreinigungsvorrichtung als Tischgerät auszubilden und mit einem leichtgewichtigen Ständer auf einem Tisch aufzustellen.

Weiteres Merkmal der Erfindung ist, dass eine solche personalisierte Luftreinigungsvorrichtung in Form eines scheibenförmigen Gehäuses, das bevorzugt rotationssymmetrisch ausgebildet ist, nunmehr die Möglichkeit bietet, dass das Gerät auf einem Tischständer in beliebiger Weise um eine horizontale Rotationsachse gedreht werden kann, um so eine gezielte Anstrahlung des Körpers des Benutzers zu ermöglichen.

Somit wird ein leicht handhabbares Gerät mit extrem verkürzten Luftführungskanälen beschrieben, denn nach einem weiteren Merkmal der Erfindung befinden sich der Lufteinsaugbereich und der Luftausblasbereich in der gleichen radialen Ebene am äußeren Umfang des Gehäuses, was mit dem Vorteil verbunden ist, dass eine sehr geringe Baubreite (Höhe der Diskusscheibe) erreicht werden kann.

Der Mittenquerbereich des etwa scheibenförmigen Gehäusekörpers spannt eine radiale Ebene auf, die sich vom Mittelpunkt des Gerätes radial nach außen in Richtung zum Außenumfang erstreckt. In dieser radialen Ebene sind nun sowohl der Lufteinsaugbereich als auch der Luftausblasbereich fluchtend und ohne axialen Versatz zueinander angeordnet, was zu einer geringen Baubreite führt.

Somit ist es möglich, an einem ersten Umfangsbereich am Außenumfang des Gehäuses einen Luftausblasstutzen anzuordnen, mit dem die gereinigte und möglicherweise auch beduftete Ausblasluft gezielt auf den Körper des Benutzers - bevorzugt auf den Atembereich im Gesicht - gerichtet werden kann und an einem zweiten Umfangsbereich am Außenumfang des Gehäuses den Lufteinsaugbereich anzuordnen. Beide Bereiche befinden sich in der gleichen, am Außenumfang des Gehäuses angeordneten Ringnut.

Dabei wird bevorzugt, wenn der Ausblasluftstrom laminar strömt und turbulenzfrei ist und in seiner Ausblasstärke einstellbar ausgebildet ist, um zu gewährleisten, dass der Benutzer ein direkt auf das Gesicht (und die Atemwege) gerichteten Luftstrom nicht als störend bemerkt.

Es sind sogar in einer Weiterbildung der Erfindung Vorkehrungen getroffen, damit der Benutzer überhaupt einen auf das Gesicht gerichteten Luftstrom feststellen kann, obwohl der Luftstrom selbst nicht bemerkbar ist.

Zu diesem Zweck ist es vorgesehen, dass am oder im Ausblasstutzen optisch wahrnehmbare Leuchtelemente vorhanden sind, die nur dann aufleuchten, wenn auch tatsächlich ein Ausblasluftstrom erzeugt wird.

Die Erfindung ist nicht auf die Ausbildung des Gehäuses als Tischgerät beschränkt. In einer anderen Ausgestaltung kann es vorgesehen sein, dass das Gerät in der gleichen oder einer im Durchmesser vergrößerten Ausführung als Wandgerät verwendet werden kann. Es wird dann an einen Wandständer gehängt und kann an diesem Wandständer ebenfalls um eine horizontale Achse drehbar ausgebildet sein.

In einer bevorzugten Ausführungsform ist vorgesehen, dass das scheibenförmige Luftreinigungsgerät auch mit einer Autositzhalterung an der Rückenlehne des Vordersitzes eines Kraftfahrzeuges befestigt wird und ebenfalls dort schwenkbar oder drehbar gelagert ist.

Allen Ausführungsformen ist gemeinsam, dass ein frei aus dem Gehäuse der Luftreinigungsvorrichtung abgestrahlter Ausblasluftstrom individuell auf den Benutzer gerichtet werden kann und dieser Ausblasluftstrom mindestens durch ein oder mehrere Filter gereinigt ist und noch zusätzliche Eigenschaften aufweisen kann.

Wie vorhin angegeben, kann noch eine zusätzliche Vorrichtung zur Beduftung des Ausblasluftstromes vorgesehen sein. Ebenso kann es vorgesehen sein, dass im Innenraum des Gerätes ein lonisator angeordnet ist, um die Abgabe von ionisierter Luft zu ermöglichen.

Demnach betrifft die Erfindung eine Luftreinigungsvorrichtung, zur personalisierten Luftzufuhr, mit einem Gehäuse, welches zumindest einen Lufteinsaugbereich zum Einsaugen von Luft in das Gehäuse und zumindest einen Luftausblasbereich zum Ausblasen der Luft aus dem Gehäuse aufweist, wobei im Gehäuse zumindest ein, um eine Ventilatorrotationsachse rotierbares Ventilatorrad der Luftreinigungsvorrichtung und zumindest ein Einsaugluftfilter der Luftreinigungsvorrichtung zum Filtern der durch den Lufteinsaugbereich eingesaugten Luft angeordnet sind und das Ventilatorrad die Luft im Betrieb der Luftreinigungsvorrichtung zumindest in einer Richtung parallel zur Ventilatorrotationsachse in das Ventilatorrad einsaugt und in zumindest einer Richtung radial zur Ventilatorrotationsachse vom Ventilatorrad wegbläst.

Mit den vorgenannten Merkmalen ergibt sich eine sehr geringe Bauhöhe (=Scheibendicke), wenn das Ventilatorrad in einem vom Einsaugluftfilter teilweise umgebenen Innenraum angeordnet ist und der Lufteinsaugbereich und der Luftausblasbereich in der gleichen radialen Ebene am äußeren Umfang des Gehäuses angeordnet sind.

Von besonderem Vorteil ist, wenn der Lufteinsaugbereich und der Luftausblasbereich, von der Ventilatorrotationsachse aus gesehen, am radial äußeren Rand des Gehäuses angeordnet sind. Damit ergibt sich die Möglichkeit, dass der Lufteinsaugbereich und der Luftausblasbereich im Bereich einer im Mittenbereich des Gehäuses am Außenumfang angeordneten, umlaufenden Ringnut angeordnet sind, was die Bauhöhe (=Scheibendicke) weiter reduziert.

Durch eine Anordnung des Ventilatorrades in einem vom Einsaugluftfilter teilweise umgebenen Innenraum und damit teilweise innerhalb des Einsaugluftfilters wird eine sehr kompakte Bauweise der Luftreinigungsvorrichtung möglich. Hierdurch können sehr einfach transportable und auch vielseitig einsetzbare Luftreinigungsvorrichtungen geschaffen werden. Damit ist es möglich, eine solche Luftreinigungsvorrichtung als Tischgerät oder als Boden- oder als Wandgerät auszubilden, was vorher nicht möglich war.

Das Ventilatorrad ist dabei das Bauteil der Luftreinigungsvorrichtung mit welchem die Luftbewegung zum Einsaugen der Luft durch den Lufteinsaugbereich, zum Transport der Luft durch das Gehäuse und zum Ausblasen der Luft durch den Luftausblasbereich aus dem Gehäuse heraus erzeugt wird. Es handelt sich dabei um ein Rad, welches im Betrieb der Luftreinigungsvorrichtung um die Ventilatorrotationsachse in einer Rotationsrichtung rotiert. Das Ventilatorrad hat in bevorzugten Ausgestaltungsformen eine Vielzahl von Luftschaufeln, welche von der Ventilatorrotationsachse, vorzugsweise distanziert, angeordnet sind oder sich zumindest in diese Richtung erstrecken.

Diese Luftschaufeln können auf einer Grundfläche des Ventilatorrades angeordnet sein und von dieser Grundfläche in Richtung parallel zur Rotationsachse, vorzugsweise orthogonal abstehen. Die Grundfläche ist in bevorzugten Ausgestaltungsformen orthogonal zur Ventilatorrotationsachse angeordnet.

Bevorzugt sind die Luftschaufeln, von der Ventilatorrotationsachse aus gesehen, abgewinkelt gegen die Radialrichtung ausgerichtet. In bevorzugten Ausgestaltungsformen ist der Ventilator bzw. das Ventilatorrad als ein sogenannter, rückwärts gekrümmter Radialventilator ausgebildet.

Die erfindungsgemäße Luftreinigungsvorrichtungen sieht jedenfalls vor, dass das Ventilatorrad die Luft im Betrieb der Luftreinigungsvorrichtung zumindest in einer Richtung parallel zur Ventilatorrotationsachse in das Ventilatorrad einsaugt und in zumindest einer Richtung radial zur Ventilatorrotationsachse vom Ventilatorrad wegbläst.

Erfindungsgemäße Luftreinigungsvorrichtungen können als Raumluftreiniger eingesetzt werden, um die Luft in einem Raum zu reinigen. Die erfindungsgemäße Luftreinigungsvorrichtung sieht aber vor, dass die gereinigte Luft gezielt in eine Richtung bzw. in einen Richtungsbereich ausgeblasen wird. Solche erfindungsgemäßen Luftreinigungsvorrichtungen können auch als sogenannte Atemzonenluftreiniger eingesetzt werden, die z.B. dazu verwendet werden können, einer Person gezielt gereinigte Luft in die Atemzone zu blasen. Damit wird der Luftreiniger einer bestimmten Person zugordnet und ist somit personalisiert.

Die Erfindung sieht vor, dass, in einer Draufsicht aus Richtung parallel zur Ventilatorrotationsachse gesehen, der Einsaugluftfilter den Innenraum, in dem das Ventilatorrad angeordnet ist, nur unvollständig umgibt.

Es ist dabei vorgesehen, dass, in der Draufsicht aus Richtung parallel zur Ventilatorrotationsachse gesehen, im vom Einsaugluftfilter frei bleibenden Abschnitt ein Ausblasraum angeordnet ist, in den das Ventilatorrad im Betrieb die Luft auf dem Weg hin zum Luftausblasbereich des Gehäuses einbläst.

Hierdurch können besonders kompakte Luftreinigungsvorrichtungen gebaut werden. Im Ausblasraum erfindungsgemäßer Luftreinigungsvorrichtungen können Ausblasluftfilter angeordnet sein. Es sind aber auch erfindungsgemäße Varianten einer Luftreinigungsvorrichtung denkbar, bei denen auf den Ausblasluftfilter im Ausblasraum verzichtet wird.

Vereinfacht gesprochen, ist es gemäß der Erfindung vorgesehen, dass der Ausblasraum und der Lufteinsaugbereich in derselben Ebene angeordnet werden. Damit ist der Vorteil einer bisher nicht gekannten Reduzierung der Bauhöhe verbunden.

Bei einer bevorzugten Variante der Erfindung ist vorgesehen, dass der Lufteinsaugbereich des Gehäuses von zwei gedachten, zueinander parallelen Begrenzungsebenen begrenzt ist und ein bzw. der Ausblasraum, in den das Ventilatorrad die Luft im Betrieb auf dem Weg hin zum Luftausblasbereich des Gehäuses einbläst, und/oder der Luftausblasbereich des Gehäuses zumindest teilweise, vorzugsweise vollständig, zwischen den parallelen Begrenzungsebenen angeordnet ist bzw. sind. Die Begrenzungsebenen stehen dabei bevorzugt orthogonal bzw. normal zur Ventilatorrotationsachse.

Insbesondere, um den Ausblasraum freizugeben, sieht die Erfindung vor, dass der Einsaugluftfilter den Innenraum, in dem das Ventilatorrad zumindest teilweise angeordnet ist, nicht vollständig umschließt. In diesem Sinne sehen bevorzugte Varianten der Erfindung vor, dass der Einsaugluftfilter C-förmig ausgebildet ist. Dabei ist der Begriff der C-förmigen Ausgestaltung weit aufzufassen. Das C kann dabei gebogen oder eckig usw. sein. Es handelt sich letztendlich um Formen des Einsaugluftfilters, welche den genannten Innenraum umgeben aber nicht vollständig umfangsgeschlossen sind.

Bevorzugte Varianten der Erfindung sehen vor, dass zwischen dem Einsaugluftfilter und dem Ventilatorrad eine Barrierewand zur Umlenkung der durch den Einsaugluftfilter hindurchgesaugten Luft hin zum Ventilatorrad angeordnet ist. Die Barrierewand ist dabei vorteilhaft ebenfalls in dem vom Einsaugluftfilter umgebenen Innenraum angeordnet.

Mit der Barrierewand wird es möglich, dass ein Luftstrom der vom Ventilatorrad durch den Lufteinsaugbereich in das Gehäuse eingesaugten Luft bezüglich der Ventilatorrotationsachse in radialer Richtung in den Einsaugluftfilter hinein und anschließend zumindest auch in Richtungen parallel zur Ventilatorrotationsachse um die Barrierewand herum hin zum Ventilatorrad verläuft. Die Barrierewand weist vorteilhaft eine Öffnung zum Einblasen der Luft vom Ventilatorrad in den bzw. einen Ausblasraum auf. Die Luft wird dabei vom Ventilatorrad in zumindest einer Richtung radial zur Ventilatorrotationsachse in den Ausblasraum eingeblasen.

Die Barrierewand kann dabei einen Aufnahmeraum für das Ventilatorrad teilweise umschließen. Bevorzugt ist das Ventilatorrad in dem Aufnahmeraum exzentrisch angeordnet. Dies bedeutet, dass das Ventilatorrad in gewissen Bereichen näher an der Barrierewand angeordnet ist, als in anderen Bereichen des Aufnahmeraums. Der Aufnahmeraum für das Ventilatorrad befindet sich, vorteilhaft zusammen mit der Barrierewand, zumindest teilweise in dem Innenraum, welcher vom Einsaugluftfilter zumindest teilweise umgeben ist.

Erfindungsgemäß ist es vorgesehen, dass das Gehäuse in einer Ansicht aus Richtung parallel zur Ventilatorrotationsachse eine kreisrunde Außenkontur aufweist. Das Gehäuse kann insgesamt eine diskusförmige Außenkontur haben. Der Lufteinsaugbereich und/oder der Luftausblasbereich können von der Ventilatorrotationsachse aus gesehen, am radial äußeren Rand des Gehäuses angeordnet sein. Ferner ist vorgesehen, dass der Lufteinsaugbereich und der Luftausblasbereich von der Rotationsachse aus gesehen, in voneinander verschiedenen Bereichen des radial äußeren Randes des Gehäuses angeordnet sind. Sowohl im Lufteinsaugbereich als auch im Luftausblasbereich kann jeweils eine Abfolge von Lamellen angeordnet sein. Zwischen den Lamellen im Lufteinsaugbereich kann Luft in das Gehäuse eingesaugt werden. Zwischen den Lamellen im Luftausblasbereich kann die Luft aus dem Gehäuse ausgeblasen werden. Die Lamellen können sowohl im Lufteinsaugbereich als auch im Luftausblasbereich gegenüber der radialen Richtung von der Ventilatorrotationsachse aus gesehen, abgewinkelt angeordnet sein.

Weitere Merkmale und Einzelheiten bevorzugter Varianten der Erfindung werden nachfolgend anhand von Ausführungsbeispielen der Erfindung erläutert.

Es zeigen:
Fig. 1: eine perspektivische Darstellung der Luftreinigungsvorrichtung
Fig. 2: eine gedrehte Darstellung nach Figur 1
Figur 3: eine schematische Darstellung einer Anwendung
Figur 4: eine perspektivische Darstellung mit weiteren Einzelheiten
Figur 5: ein Schnitt gemäß der Linie X-X in Figur 4
Figur 6: eine perspektivische Zusammenstellungszeichnung
Figur 7: eine andere perspektivische Darstellung
Figur 8: eine Stirnansicht auf die Ausblasseite mit einer ersten Ausführung eines Tischständers
Figur 9: eine Stirnansicht auf die Ausblasseite mit einer zweiten Ausführung eines Tischständers
Figur 10: die Darstellung nach Figur 8 und 9 ohne Ständer
Figur 11: eine Seitenansicht
Figur 12: eine Stirnansicht auf die Ansaugseite
Figur 13: ein erster Querschnitt durch das Gerät
Figur 14: ein zweiter Querschnitt durch das Gerät
Figur 15: ein erster Längsschnitt durch das Gerät
Figur 16: ein zweiter Längsschnitt durch das Gerät
Figur 17: ein dritter Längsschnitt durch das Gerät
Figur 18: ein dritter Querschnitt durch das Gerät
Figur 19: eine perspektivische Darstellung des verwendeten Filters

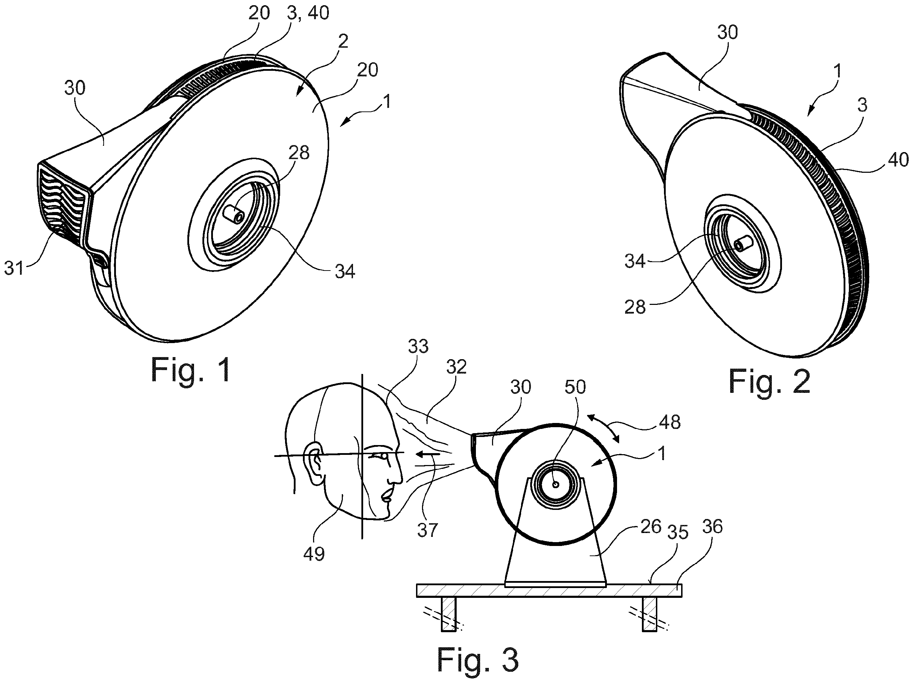

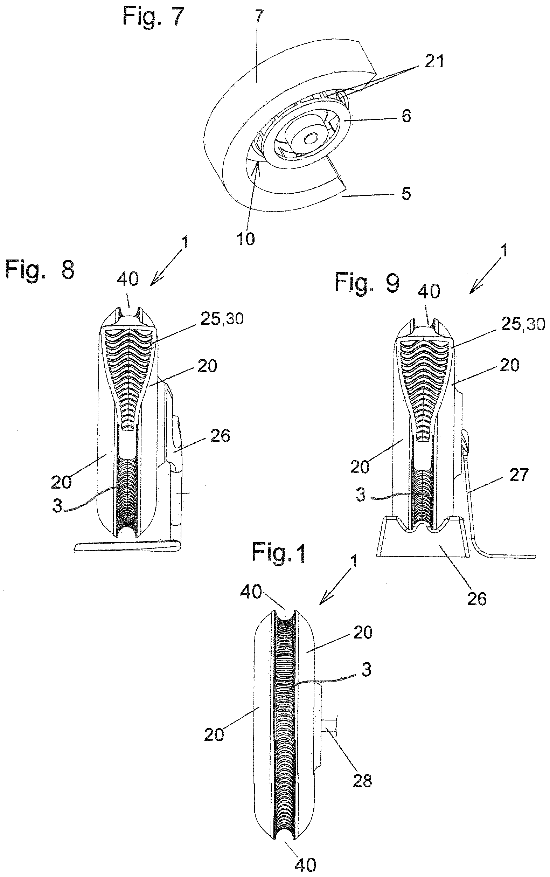

In Figur 1 ist eine perspektivische Seitenansicht der erfindungsgemäßen Luftreinigungsvorrichtung 1 in Form eines rotationssymmetrisch kreiszylindrischen Gehäuses 2 dargestellt, welches zwei zueinander symmetrische Halbschalen 20 aufweist, die zwischen sich im Mittenbereich eine mittige Ringnut 40 ausbilden, wobei bevorzugt die Ringnut 40 vertieft umlaufend im Gehäuse angeordnet ist, wie dies beispielsweise aus Figur 8 und 9 entnehmbar ist.

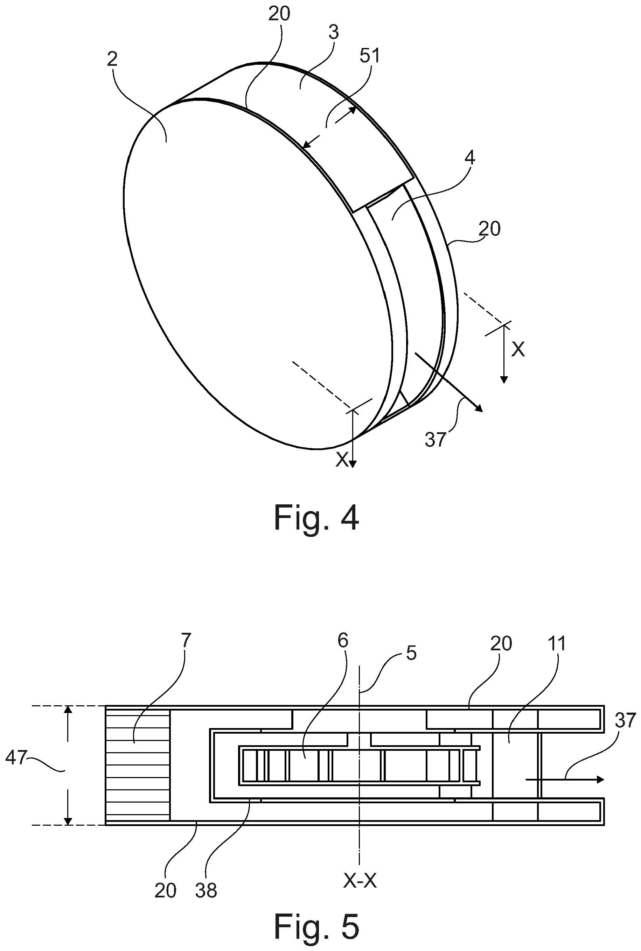

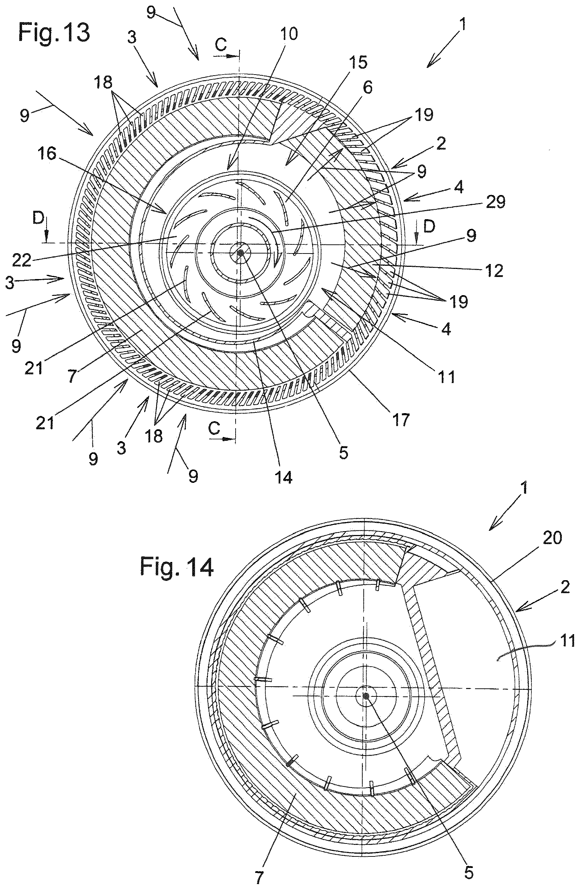

Im Bereich dieser vertieft angeordneten, mittleren Ringnut 40 sind gemäß den Figuren 4 bis 10 eine Anzahl von Lamellen 18, 19 (siehe Figur 13) angeordnet, wobei die Lamellen 18 dem Lufteinsaugbereich 3 und die Lamellen 19 dem Luftausblasbereich 4 zugeordnet sind.

Gemäß Figur 1 und 2 kann auf den Luftauslassbereich 4 nunmehr ein Ausblasstutzen 30 aufgesteckt werden, der eine etwa trompetenförmige Gestalt aufweist und in seinem vorderen Bereich ein Ausströmgitter 31 aufweist, welches eine laminare Luftströmung über den Ausblasstrom 32 (siehe Figur 3) erzeugt.

Somit strömt aus dem Ausströmgitter 31 des Ausblasstutzens 30 ein bevorzugt laminarer Ausblasluftstrom 32, der in Pfeilrichtung 37 z. B. gegen den Kopfbereich 33 eines Benutzers 49 gerichtet werden kann.

Besonders bevorzugt wird es, wenn der Ausblasluftstrom 32 direkt in den Atembereich des Benutzers 49 gerichtet ist, so dass der Benutzer 49 vorwiegend gereinigte Ausblasluft aus dem Ausblasstutzen 30 empfängt, die z. B. von Giftstoffen, Fremdgerüchen, Pflanzen- und Gräserpollen und dergleichen gereinigt ist.

Die Figur 3 zeigt auch noch, dass die Luftreinigungsvorrichtung etwa scheibenförmig in der Art einer Diskusscheibe ausgebildet ist, wie sie beispielsweise als Wurfscheibe in der Leichtathletik verwendet wird. Auch die Größenverhältnisse der Ausführung nach den Figuren 1 bis 3 entsprechen denen einer Wurfscheibe.

Damit ist es erstmals möglich, eine solche Luftreinigungsvorrichtung 1 gemäß Figur 3 auf einem Standfuß 26 in den Pfeilrichtungen 48 drehbar anzuordnen, um eine freie Drehbarkeit um die Drehachse 50 des Gehäuses 2 zu erreichen, wobei im Bereich dieser Drehachse 50 gleichzeitig auch die Stecköffnung 34 für einen Stromanschluss angeordnet ist, dessen Stromkabel in die geräteseitige Anschlussbuchse 28 drehbar eingreift.

Die Figuren 4 und 5 zeigen weitere Einzelheiten des Gerätes nach den Figuren 1 bis 3, und es ist gut erkennbar, dass bei einem rotationssymmetrischen Gehäuse 2 gemäß Figur 4 sich der Lufteinsaugbereich 3 etwa C-förmig um den Außenumfang des Gehäuses 2 erstreckt und deshalb in diesem großflächigen Bereich ein daran angepasster C-förmiger Einsaugluftfilter 7 (siehe Figur 7 und Figur 14) angeordnet werden kann, so dass ein hoher Volumenstrom durch ein eine große Filterfläche aufweisenden Einsaugluftfilter 7 gefiltert werden kann.

Würde die Luft hingegen in axialer Richtung der Ventilatorrotationsachse angesaugt werden, das heisst, im Bereich einer der Deckflächen des scheibenförmigen Gehäuses, wäre es nicht möglich, in diesem Bereich einen großflächigen Luftfilter anzuordnen, ohne die Baubreite des Gerätes wesentlich zu erhöhen.

Somit liegt der erfinderische Verdienst der Erfindung darin, den Luftansaugbereich in den Umfangsbereich am Außenumfang des Gerätes zu legen, um direkt dahinter im Gehäuse einen großflächigen bevorzugt als Faltenfilter ausgebildeten-Einsaugluftfilter 7 anzuordnen zu können, der die Ansaugluft über einen weiten Umfangsbereich ansaugt und einen konisch zum Ventilatorrad 6 zulaufenden Luftstrom im Gehäuse erzeugt.

Während sich der Lufteinsaugbereich 3 um einen Umfangswinkel von z. B. 270° um das Gehäuse 2 herum erstreckt, beträgt der Winkel des Luftausblasbereiches 4 nur noch einen Winkel von z. B. 90°.

Aus Figur 5 sind weitere Einzelheiten der Anordnung zu entnehmen, wobei wichtig ist, dass sowohl der Lufteinsaugbereich 3 am Außenumfang des Gehäuses 2 im Bereich der vorher erwähnten radialen Ringnut 40 liegt, als auch der Luftausblasbereich 4.

Weil die beiden Bereiche 3, 4 in der gleichen horizontalen Ebene 39 des Gehäuses 2 liegen, ergibt sich eine besondere geringe Baubreite 47 des Gehäuses 2, was eine geringen Scheibendicke des rotationssymmetrischen Gehäuses 2 entspricht.

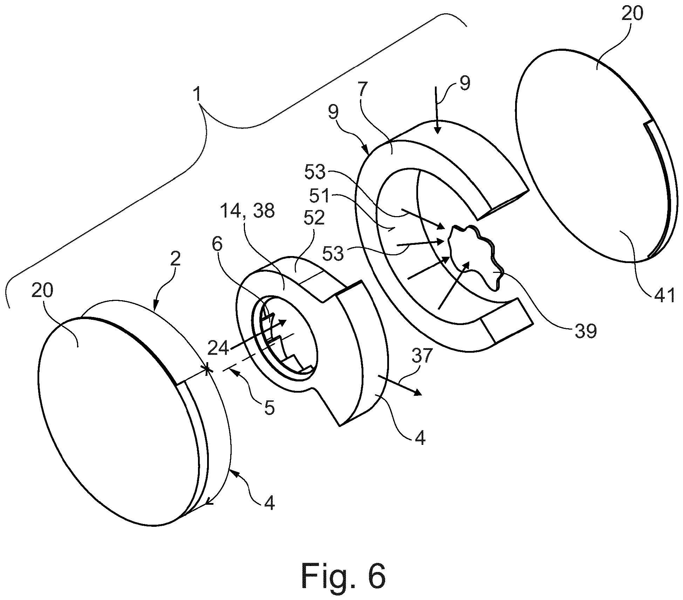

Die Figur 6 zeigt den Prinzipaufbau des Gehäuses 2, wo erkennbar ist, dass die beiden im Wesentlichen gleich ausgebildeten Halbschalen 20 zwischen sich ein Luftführungsgehäuse 38 aufnehmen, in dessen zentralen Innenraum das Ventilatorrad 6 aufgenommen ist, das um die Ventilatorrotationsachse 5 drehend angetrieben ist.

Ferner ist aus Figur 6 der C-förmige Einsaugluftfilter 7 zu entnehmen, der an seiner Innenseite den C-förmigen Reinluftbereich 51 ausbildet, der einen radial nach innen gerichteten Reinluftstrom in Richtung auf den Luftzuführungsbereich 51 des Luftführungsgehäuses 38 bildet. Somit wird durch die C-Form ein besonders großflächiger Reinluftstrom 53 an der radialen Innenseite des Einsaugluftfilters 7 gebildet, der in Richtung radial einwärts auf das Ventilatorrad 6 komprimiert wird.

In der gleichen Ebene 39 des Einsaugluftfilters 7 ist auch der Luftausblasbereich 4 des Luftführungsgehäuses 38 angeordnet, so dass die genannten Teile in der gleichen Ebene 39 angeordnet sind.

Aus den Figuren 7 bis 10 ergeben sich weitere Einzelheiten des Aufbaus des Gehäuses 2 mit den luftführenden Elementen.

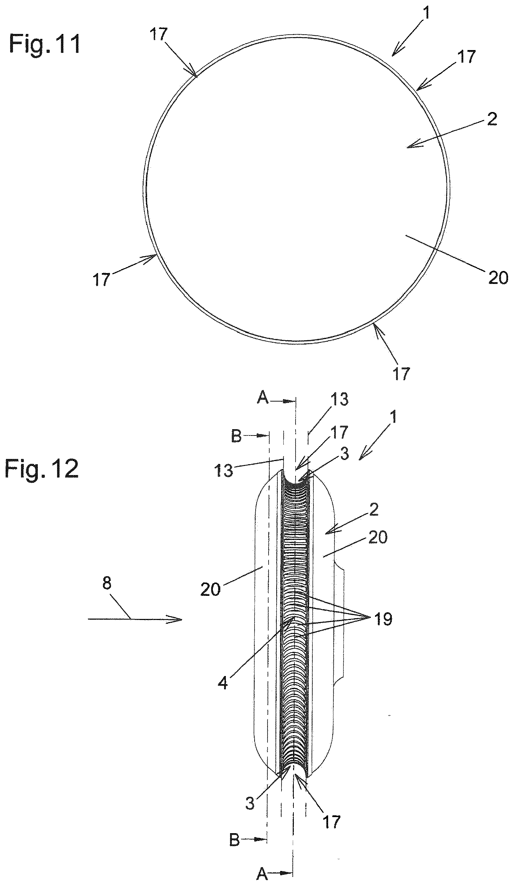

In der Ansicht aus Richtung 8 parallel zur Ventilatorrotationsachse 5 gemäß Fig. 11 weist das Gehäuse 2 eine kreisrunde Außenkontur auf. Betrachtet man die Seitenansicht gemäß Fig. 12 auf die Luftreinigungsvorrichtung 1 aus einer hierzu orthogonalen Richtung, so erkennt man, dass in diesem Ausführungsbeispiel das Gehäuse 2 eine diskusförmige Außenkontur aufweist. Der Lufteinsaugbereich 3 und/oder der Luftausblasbereich 4 sind am radial äußeren Rand 17 des Gehäuses 2 angeordnet. Sie bilden gemeinsam einen um das Gehäuse 2 umlaufenden Bereich von Öffnungen für den Lufteintritt und -austritt in und aus dem Gehäuse. Im gezeigten Ausführungsbeispiel ist dies an den Lamellen 18 und 19 zu erkennen, die sich im Lufteinsaugbereich 3 und im Luftausblasbereich 4 befinden, wie dies insbesondere in dem Schnitt entlang der Schnittlinie A-A aus Fig. 12 in Fig. 13 zu erkennen ist.

Dort ist auch gut zu sehen, dass der Lufteinsaugbereich 3 und der Luftausblasbereich 4 in von der Ventilatorrotationsachse 5 aus gesehen voneinander verschiedenen Bereichen des radial äußeren Randes 17 des Gehäuses 2 angeordnet sind. In Fig. 12 ist gut zu sehen, dass sich zwischen den Halbschalen 20 des Gehäuses 2 am Rand 17 die Lamellen 18 und 19 und damit auch der Lufteinsaugbereich 3 und der Luftausblasbereich 4 befinden.

In Fig. 12 sind auch die beiden zueinander parallelen Begrenzungsebenen 13 eingezeichnet. Sowohl der Lufteinsaugbereich 3 als auch der Luftausblasbereich 4 dieses Ausführungsbeispiels sind von diesen zueinander parallelen Begrenzungsebenen 13 begrenzt. Auch der weiter unten noch erwähnte Ausblasraum 11, in den das Ventilatorrad 6 die Luft im Betrieb auf dem Weg hin zum Luftausblasbereich 4 des Gehäuses einbläst, befindet sich zwischen diesen beiden zueinander parallelen Begrenzungsebenen 13. Die beiden gedachten Begrenzungsebenen 13 sind normal bzw. orthogonal zur Richtung 8 und damit auch zum Längsverlauf der Ventilatorrotationsachse 5 angeordnet.

In dem Schnitt entlang der Schnittlinie A-A aus Fig.12, wie er in Fig. 13 gezeigt ist, ist gut zu erkennen, dass das Ventilatorrad 6 erfindungsgemäß in einem vom Einsaugluftfilter 7 zumindest teilweise umgebenen Innenraum 10 angeordnet ist. Hierbei ist vorgesehen, dass in der in Fig. 13 gezeigten Draufsicht aus Richtung 8 parallel zur Ventilatorrotationsachse 5 gesehen, der Einsaugluftfilter 7 den Innenraum 10, in dem das Ventilatorrad 6 angeordnet ist, nur unvollständig umgibt.

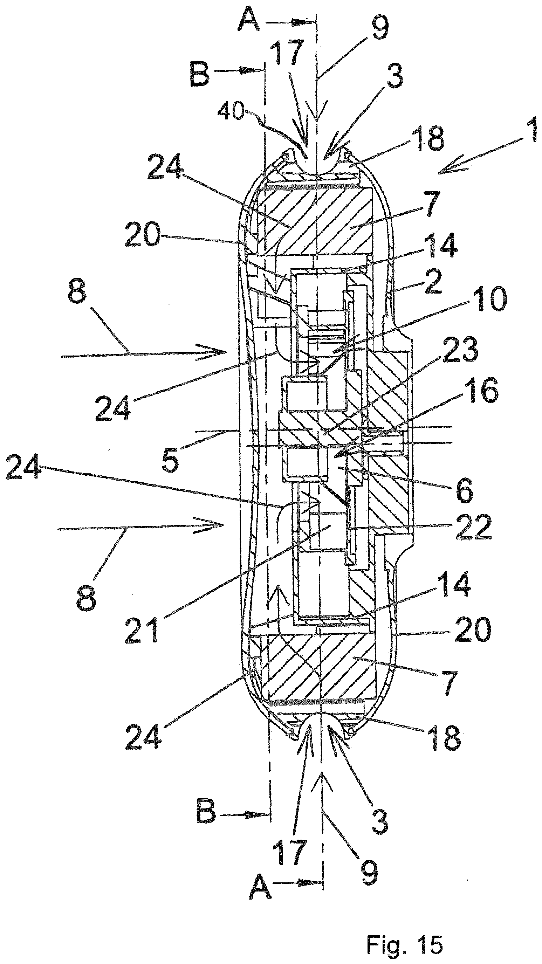

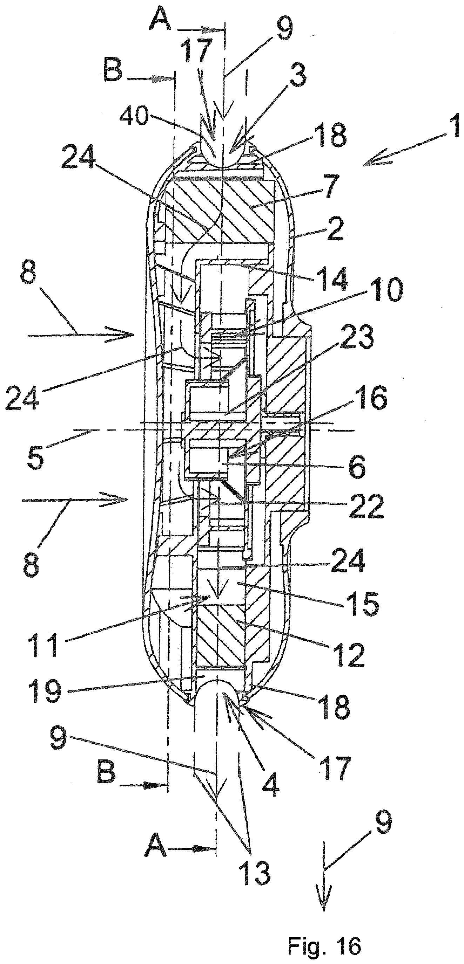

Im vom Einsaugluftfilter 7 freibleibenden Abschnitt ist der Ausblasraum 11 angeordnet, in den das Ventilatorrad 6 im Betrieb die Luft auf dem Weg hin zum Luftausblasbereich 4 des Gehäuses 2 einbläst. In dem Ausblasraum 11 ist im ersten Ausführungsbeispiel, wie dies besonders gut in Fig. 13 zu sehen ist, ein Ausblasluftfilter 12 angeordnet. Der Einsaugluftfilter 7 dieses Ausführungsbeispiels ist C-förmig ausgebildet. Zwischen dem Einsaugluftfilter 7 und dem Ventilatorrad 6 befindet sich die Barrierewand 14. Mit dieser wird die durch den Einsaugluftfilter 7 hindurch eingesaugte Luft auf dem Weg hin zum Ventilatorrad 6 umgelenkt, wie dies weiter unten noch im Detail anhand der Fig.15 und 16 gezeigt ist.

In Fig. 13 sind die Richtungen 9 radial zur Ventilatorrotationsachse 5 eingezeichnet. In diesen Richtungen 9 wird die zu reinigende Luft im Lufteinsaugbereich 3 mittels des um seine Ventilatorrotationsachse 5 rotierenden Ventilatorrades 6 angesaugt. Die angesaugte Luft durchdringt die Zwischenräume zwischen den Lamellen 18 des Gehäuses 2 im Lufteinsaugbereich 3 und strömt dann in radialer Richtung 9 in den Einsaugluftfilter 7. Die Barrierewand 14 lenkt dann die so eingesaugte Luft auf dem Weg vom Einsaugluftfilter 7 hin zum Ventilatorrad 6 um. Die Luft wird dann in Richtung 8 parallel zur Ventilatorrotationsachse 5 in das Ventilatorrad 6 eingesaugt und in Richtung 9 radial nach außen vom Ventilatorrad 6 durch eine Öffnung 15 in der Barrierewand 14 hindurch in den Ausblasraum 11 ausgeblasen. Die Luft verlässt den Ausblasraum 11 in diesem Ausführungsbei spiel dann durch den Ausblasluftfilter 12 und durch die Lamellen 19 des Luftausblasbereichs 4 hindurch.

In Fig. 13 ist auch gut zu erkennen, dass sowohl die Lamellen 18 als auch die Lamellen 19 in diesem Ausführungsbeispiel jeweils abgewinkelt zur radialen Richtung 9 angeordnet sind.

Die Barrierewand 14 umschließt den Aufnahmeraum 16, in dem sich das Ventilatorrad 6 befindet, teilweise. In Fig. 13 ist gut zu erkennen, dass das Ventilatorrad 6 in diesem Aufnahmeraum 16 exzentrisch angeordnet ist. Es befindet sich somit auf der einen Seite, nämlich in Fig. 13 links unten, näher an der Barrierewand 14 als auf der gegenüberliegenden Seite, in Fig. 13 links oben.

Um den genannten Luftstrom zu erzeugen, rotiert das Ventilatorrad 6 um seine Ventilatorrotationsachse 5. Es wird hierzu von dem in den Fig. 15 und 16 eingezeichneten Ventilatormotor 23 gedreht. Die Rotationsrichtung 29 des sich im Betrieb befindlichen Ventilatorrades 6 ist in Fig. 13 eingezeichnet.

Im gezeigten Ausführungsbeispiel handelt es sich beim Ventilatorrad 6 bzw. beim Ventilator um einen sogenannten rückwärts gekrümmten Radialventilator. Dies ist auch gut an der Form der Luftschaufeln 21 zu erkennen. Diese sind auf einer Grundfläche 22 des Ventilatorrades 6 angeordnet und erstrecken sich in eine Richtung 8 parallel zur Ventilatorrotationsachse 5. In einer nicht zeichnerisch dargestellten Variante kann der Ventilator auch als vorwärtsgekrümmter Radialventilator ausgebildet sein.

Als Einsaugluftfilter 7 können unterschiedlichste Filter zum Einsatz kommen. Z.B. kann es sich um Papierfilter, insbesondere gefaltete Papierfilter, handeln, um Staub bzw. Partikel aus der Luft herauszufiltern. Es kann sich aber auch um andere poröse Materialien mit einer entsprechenden Luftreinigungswirkung handeln. Das Gleiche gilt für den gegebenenfalls vorhandenen Ausblasluftfilter 12. Beide Filtertypen können z.B. auch Aktivkohle oder dergleichen zur Beseitigung von Keimen und dergleichen aufweisen.

Fig. 14 zeigt einen Schnitt in der Schnittebene B-B gemäß Fig. 12. Diese Schnittebene B-B verläuft parallel zur Schnittebene A-A in einem Bereich, in dem sich weder die Barrierewand 14 noch die Luftschaufeln 21 des Ventilatorrades 6 befinden, also in dem Bereich, in dem die durch den Einsaugluftfilter 7 hindurchgesaugte Luft die Barrierewand 14 passieren kann. Auch der in diesem Ausführungsbeispiel vorhandene Ausblasluftfilter 12 ist in dieser Schnittebene B-B nicht mehr vorhanden.

Fig. 15 zeigt einen Schnitt durch die Luftreinigungsvorrichtung 1 dieses ersten Ausführungsbeispiels, in der in Fig. 13 eingezeichneten Schnittebene C-C. Diese verläuft parallel zur Ventilatorrotationsachse 5 und schneidet den Einsaugluftfilter 7 zweimal.

Fig. 16 zeigt einen Schnitt durch diese Luftreinigungsvorrichtung 1 in der ebenfalls in Fig. 13 eingezeichneten Schnittebene DD. Auch die Schnittebene DD verläuft parallel zur Ventilatorrotationsachse 5. Sie ist allerdings orthogonal zur Schnittebene CC angeordnet und schneidet einerseits den Lufteinsaugbereich 3 mit dem Einsaugluftfilter 7 und andererseits aber auch den Luftausblasbereich 4 und den hier in diesem Ausführungsbeispiel angeordneten Ausblasluftfilter 12.

Anhand der Fig. 15 und 16 ist die Luftführung im Gehäuse 2 besonders gut nachvollziehbar. Der Luftstrom 24 ist schematisiert in Form von Pfeilen in den Fig. 15 und 6 eingezeichnet. In den Fig. 15 und 16 ist anhand der den Luftstrom 24 symbolisierenden Pfeile gut zu erkennen, dass die Luft von außen im Lufteinsaugbereich 3 in radialer Richtung 9 hin zur Ventilatorrotationsachse 5 in den Einsaugluftfilter 7 eingesaugt wird.

Mittels der Barrierewand 14 erfolgt dann innerhalb des Gehäuses 2 eine Umlenkung des Luftstroms 24 der eingesaugten Luft mit Bewegungskomponenten auch in Richtungen 8 parallel zur Ventilatorrotationsachse 5. Sobald die eingesaugte Luft die Barrierewand 14 passiert hat, wird sie dann in Richtung 8 parallel zur Ventilatorrotationsachse 5 in das Ventilatorrad 6 eingesaugt. Das mittels des Ventilatormotors 23 in Rotationsrichtung 29 rotierende Ventilatorrad 6 drückt dann mit seinen Luftschaufeln 21 die Luft durch die Öffnung 15 in der Barrierewand 14 in Richtung 9 radial nach außen in den Ausblasraum 11. In diesem befindet sich, wie dies in Fig. 16 unten zu sehen ist, in diesem Ausführungsbeispiel der Ausblasluftfilter 12. Durch diesen hindurch wird die Luft vom Ventilatorrad 6 in radialer Richtung 9 nach außen, also weg von der Ventilatorrotationsachse 5 geblasen, sodass sie durch den Luftausblasbereich 4 das Gehäuse 2 verlässt.

Kurz zusammengefasst wird also die zu reinigende Luft in radialen Richtungen 9 in das Gehäuse 2 eingesaugt, dann in zur Ventilatorrotationsachse 5 parallelen Richtung 8 um die Barrierewand 14 herumgelenkt und in das Ventilatorrad 6 eingesaugt und von dort in radialen Richtungen 9 nach außen von der Ventilatorrotationsachse 5 durch den Ausblasraum 11 und den Luftausblasbereich 4 als gereinigte Luft wieder aus dem Gehäuse 2 ausgeblasen.

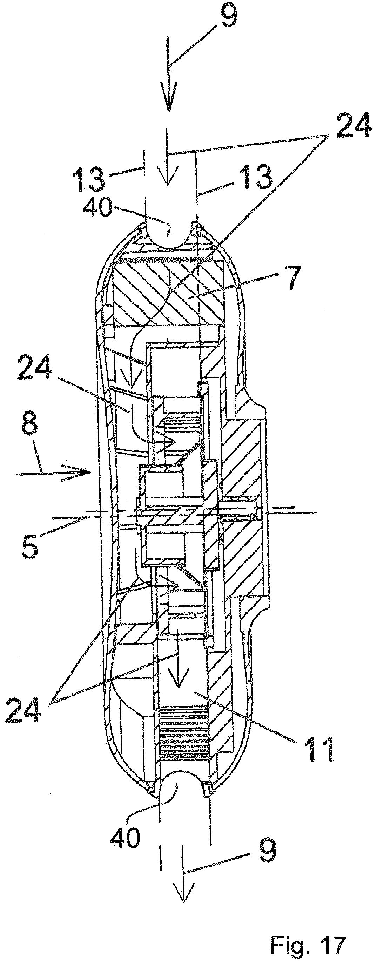

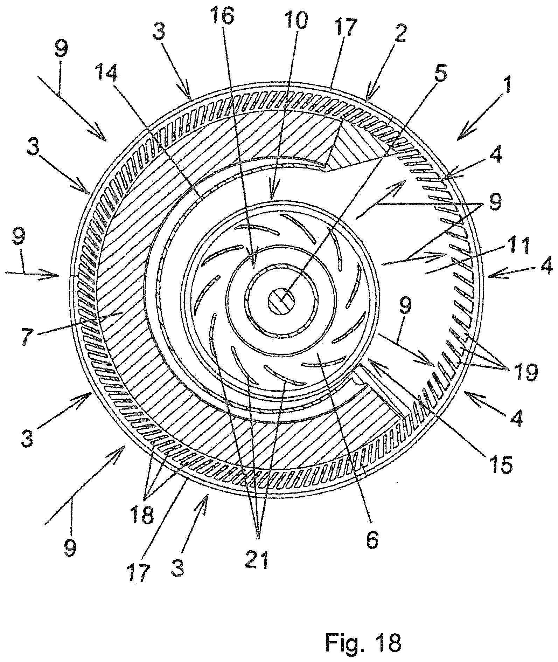

In den Fig. 17 und 18 ist ein zweites Ausführungsbeispiel der Erfindung dargestellt, bei dem auf den Ausblasluftfilter 12 verzichtet wurde. Ansonsten entspricht dieses zweite Ausführungsbeispiel dem ersten Ausführungsbei spiel, sodass auf die obigen Ausführungen hierzu verwiesen werden kann.

Die Fig. 18 zeigt einen zu Fig. 13 analogen Schnitt. Der Schnitt gemäß Fig. 17 entspricht dem Schnitt gemäß Fig. 16 des ersten Ausführungsbeispiels. In diesem zweiten Ausführungsbeispiel gemäß der Fig.17 und 18 erfolgt die Reinigung der Luft also ausschließlich im Einsaugluftfilter 7. Der Ausblasraum 11 dieses Ausführungsbeispiels ist leer, sodass die aus dem Gehäuse 2 durch den Luftausblasbereich 4 ausgeblasene Luft im Ausblasraum 11 nicht noch einmal mittels eines Ausblasluftfilters 12 zusätzlich gereinigt wird. Im Bereich dieses Ausblasluftfilters 12 kann noch zusätzlich eine Beduftungsvorrichtung angeordnet sein.

In Fig. 7 sind das Ventilatorrad 6 mit seiner Ventilatorrotationsachse 5 und der Einsaugluftfilter 7 der ersten beiden Ausführungsbeispiele ohne die sonstigen Bauteile der Luftreinigungsvorrichtung 1 dargestellt. Hier ist besonders gut zu erkennen, wie das Ventilatorrad 6 in dem vom Einsaugluftfilter 7 teilweise umgebenden Innenraum 10 angeordnet ist.

Die Fig. 8 bis 10 zeigen verschiedene Varianten, wie die Luftreinigungsvorrichtung 1 im Betrieb aufgestellt bzw. gehalten sein kann. In Fig. 8 wird hierzu ein Standfuß 26 seitlich an einer der Halbschalen 20 des Gehäuses 2 befestigt. In diesen Standfuß 26 kann auch gleich ein Stromanschluss für den Ventilatormotor 23 integriert sein.

Fig. 9 zeigt eine Variante, bei der das Gehäuse 2 der Luftreinigungsvorrichtung 1 in einen entsprechenden Standfuß 26 hineingestellt wird. Zur Stromversorgung des Ventilatormotors 23 wird hier ein gesondertes Stromkabel 27 in das Gehäuse 2 eingesteckt. In Fig. 10 wird seitlich an einer der Halbschalen 20 ein Adapterarm 28 befestigt. Dieser kann mit beliebigen Halterungen an verschiedensten Trägern befestigt sein. In allen Ausführungsbeispielen handelt es sich vorteilhaft um lösbare Verbindungen zwischen den Standfüßen 26 bzw. dem Adapterarm 28 und dem Gehäuse 2.

In den Fig. 8 und 9 ist zusätzlich am Luftausblasbereich 4 jeweils noch ein Formungselement 25 lösbar befestigt, mit dem die durch den Luftausblasbereich 4 ausgeblasene gereinigte Luft noch besser gezielt auf einen bestimmten Bereich konzentriert werden kann. Dies bietet sich vor allem dann an, wenn die Luftreinigungsvorrichtung 1 als Atemluftreiniger dazu verwendet wird, gezielt gereinigte Luft in den Atembereich einer Person zu lenken.

Dieses Formungselement 25 ist als gekrümmter, dem Außenumfang des Gehäuses 2 angepasster Ausblasstutzen 30 ausgebildet. Das Ausströmende ist durch eine Anzahl von gekrümmten Lamellen gebildet, die einen laminaren Ausblasluftstrom 32 in den Atembereich des Benutzers 49 (siehe Fig. 3) richten. Der Ausblasluftstrom 32 ist turbulenzarm und wird deshalb vom Benutzer 49 nicht oder kaum wahrgenommen. Es ist deshalb zusätzlich vorgesehen, dass im Luftausblasbereich 4 und/oder am Ausblasstutzen 30 eine oder mehrere farbige LED angeordnet sind, welche die Luftstromstärke und/oder deren Zusammensetzung (Beduftung, Ionisierung) optisch wahrnehmbar anzeigen.

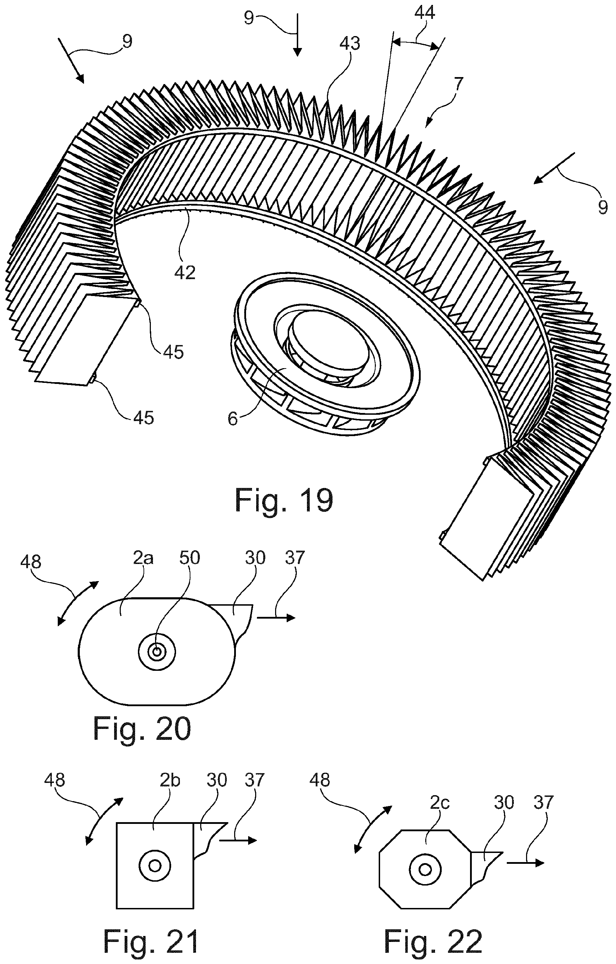

Die Figur 19 zeigt eine bevorzugte Ausführungsform eines Einsaugluftfilters 7, der aus einem Faltenfilter besteht, wobei eine Anzahl von Filterfalten 43 abgedichtet auf einem plattenförmigen C-förmigen Ringträger 42 aufgebracht sind und die obere Seite der Filterfalten durch einen alle Filterfalten innenseitig verbindenden Haltering 45 fixiert sind.

Von besonderem Vorteil ist, dass der Einsaugluftfilter 7 als Faltenfilter ausgebildet ist und sich die Filterfalten 43 konisch nach außen in einem Konuswinkel 44 öffnen.

Damit wird für die in Pfeilrichtung 9 anströmende Luft eine besonders große Filterfläche zur Verfügung gestellt und etwaige Fremdkörper fallen aufgrund des Konuswinkels 44 selbsttätig nach unten und verstopfen nicht das Einsaugluftfilter 7.

Statt eines C-förmigen Einsaugluftfilters 7 können auch andere Einsaugluftfilter verwendet werden, insbesondere Einsaugluftfilter, die über ihre gekrümmte Länge hinweg unterbrochen sind. Sie bilden dann keinen durchgehenden C-förmigen Körper, sondern sind sektorförmig aus einzelnen gekrümmten Elementen zusammengesetzt, die insgesamt dann einen C-förmigen Einsaugluftfilter 7 bilden.

Die Figuren 20 bis 22 zeigen schematisiert von der Gehäuseform des Gehäuses 2 abweichende Gehäuseformen 2a, 2b, 2c.

Die Figur 20 zeigt, dass das rotationssymmetrische Gehäuse 2 nach den Figuren 1 bis 3 auch als ovales Gehäuse 2a ausgebildet sein kann, welches um die horizontale Drehachse 50 in den Pfeilrichtungen 48 drehbar auf einem geeigneten Ständer, einer Halterung oder dergleichen gelagert ist.

In Abweichung dazu zeigt die Figur 21, dass das Gehäuse 2b auch quadratisch ausgebildet sein kann und in Figur 22 ist dargestellt, dass das Gehäuse 2c auch einen polygonalen Querschnitt aufweisen kann.

Allen Ausführungsformen ist gemeinsam, dass das Gehäuse 2, 2a-2c annähernd scheibenförmig ausgebildet ist, d. h. die Baubreite 47 hat ein Verhältnis zum Durchmesser des Gerätes im Bereich zwischen 1:2 bis 1:10.

Ebenso zeigt die Figur 3 die Verbindung mit den Figuren 20 bis 22, dass ein solches Gehäuse 2, 2a-2c einen geeigneten Standfuß 26 auf der Aufstellfläche 35 eines Tisches 36 aufgestellt werden kann. Er kann auch mit einer Wandhalterung an einer Gebäudewand drehbar um die Drehachse 50 befestigt sein oder es kann mit einem geeigneten Halter auch in einem Kraftfahrzeug angeordnet sein.

Die folgende Erfindungsbeschreibung bezieht sich auf einen Raumluftreiniger, für den die gleichen Merkmale und Vorteile gelten, wie sie vorstehend für einen Luftreiniger als Tischgerät beschrieben wurden:
- 1: Das Grundprinzip der Anordnung des Ventilators ganz oder teilweise innerhalb eines oder mehrerer umgebender Filter optional mit auf gleicher umlaufender Ebene angeordnetem Lufteinlass und Luftauslass, ist nicht nur bei einer personalisierten Luftreinigungsvorrichtung für den Tisch vorteilhaft und neuartig, sondern auch bei einem Raumluftreiniger, der durchaus auf dem Boden stehen kann und bis zu 100 cm im Durchmesser erreichen kann und ohne Ausblasstutzen auskommt.
- 2: Bei einem Raumluftreiniger resultiert aus der Anordnung des Ventilators innerhalb eines umlaufenden Filters auf gleicher Ebene, auch eine besonders flache Scheibe, die in vielen Wohn- und Arbeitsräumen von Vorteil ist. So eignet sich ein flaches Raumluftreinigungsgerät besonders dafür, gegen eine Wand gestellt zu werden oder an eine Wand gehängt zu werden. Die flache Bautiefe nimmt somit weniger aktiven Lebens- oder Arbeitsraum in Anspruch, als alternative Konzepte.
- 3: Bei einem Raumluftreiniger, erlaubt der umlaufende Lufteinlass und Luftauslass, dass die vordere und hintere Halbschale frei von optischen oder funktionalen Beeinträchtigung durch Luftein- oder Luftauslässe bleiben und so für Funktionen wie die Befestigung an einem Bodenständer oder einer Wandaufhängung genutzt werden können. Des Weiteren sind besonders Sichtflächen ohne Luftein- und -auslässe optisch attraktiv, verschmutzen nicht so schnell und können auch für gestalterische Massnahmen (aufdrucken von Bildern etc.) genutzt werden.
- 4: Die besondere Ausbildung des Filters nach Figur 19 gilt sowohl für ein Tischgerät als auch für ein Wand- oder Bodengerät. Diese besondere Ausbildung des Filters als Faltenfilter mit Falten parallel zur Rotationsachse des Ventilators und ganz oder teilweise überlappend mit dem Ventilator hat den Vorteil der Erreichung einer maximalen Filterfläche bei kleinstem Raumbedarf.

### Legende

zu den Hinweisziffern:
- 1: Luftreinigungsvorrichtung
- 2: Gehäuse
- 3: Lufteinsaugbereich
- 4: Luftausblasbereich
- 5: Ventilatorrotationsachse
- 6: Ventilatorrad
- 7: Einsaugluftfilter
- 8: Richtung
- 9: Richtung
- 10: Innenraum
- 11: Ausblasraum
- 12: Ausblasfilter
- 13: Begrenzungsebene
- 14: Barrierewand
- 15: Öffnung
- 16: Aufnahmeraum
- 17: äußerer Rand
- 18: Lamelle
- 19: Lamelle
- 20: Halbschale
- 21: Luftschaufel
- 22: Grundfläche
- 23: Ventilatormotor
- 24: Luftstrom
- 25: Formungselement
- 26: Standfuß
- 27: Stromkabel
- 28: Adapterarm
- 29: Rotationsrichtung
- 30: Ausblasstutzen
- 31: Ausströmgitter
- 32: Ausblasluftstrom
- 33: Kopfbereich
- 34: Stecköffnung
- 35: Aufstellfläche
- 36: Tisch
- 37: Pfeilrichtung
- 38: Luftführungsgehäuse
- 39: Ebene
- 40: Ringnut
- 41: Innenseite
- 42: Ringträger
- 43: Filterfalte
- 44: Konuswinkel
- 45: Haltering
- 46:
- 47: Baubreite
- 48: Pfeilrichtung
- 49: Benutzer
- 50: Drehachse
- 51: Reinluftbereich
- 52: Luftzuführungsbereich
- 53: Reinluftstrom

## Patentansprüche

1. Luftreinigungsvorrichtung (1), zur personalisierten Luftzufuhr mit einem Gehäuse (2), welches zumindest einen Lufteinsaugbereich (3) zum Einsaugen von Luft in das Gehäuse (2) und zumindest einen Luftausblasbereich (4) zum Ausblasen der Luft aus dem Gehäuse (2) aufweist, wobei im Gehäuse (2) zumindest ein, um eine Ventilatorrotationsachse (5) rotierbares Ventilatorrad (6) und zumindest ein Einsaugluftfilter (7) zum Filtern der durch den Lufteinsaugbereich (3) eingesaugten Luft angeordnet sind, wobei ein gereinigte Ausblasluftstrom (32) frei aus dem Gehäuse der Luftreinigungsvorrichtung ausgeblasen wird und individuell gegen den Körper, und insbesondere gegen das Gesicht, eines Benutzers ausrichtbar ist, wobei das Gehäuse (2) der Luftreinigungsvorrichtung (1) eine scheibenförmige Ausbildung aufweist, und der Lufteinsaugbereich (3) und der Luftausblasbereich (4) in der gleichen radialen Ebene (39) am Umfang des Gehäuses (2) angeordnet sind, **dadurch gekennzeichnet,**
**dass** das Gehäuse (2) der Luftreinigungsvorrichtung (1) in einer Ansicht aus Richtung (8) parallel zur Ventilatorrotationsachse (5) eine kreisrunde oder ovale oder polygonale Außenkontur aufweist, dass
das Ventilatorrad (6) die Luft im Betrieb der Luftreinigungsvorrichtung (1) zumindest in einer Richtung (8) parallel zur Ventilatorrotationsachse (5) in das Ventilatorrad (6) einsaugt und den gereinigten Ausblasluftstrom (32) in zumindest einer Richtung (37) radial zur Ventilatorrotationsachse (5) vom Ventilatorrad (6) wegbläst, dass
die Ventilatorrotationsachse (5) senkrecht zur Richtung (9) der radialen Ebene des Gehäuses (2) angeordnet ist, und dass das Ventilatorrad (6) in einem vom Einsaugluftfilter (7) oder mehreren Einsaugluftfiltern nur teilweise umgebenen Innenraum (10) angeordnet ist, und in der Draufsicht aus Richtung (8) parallel zur Ventilatorrotationsachse (5) gesehen, in einem vom Einsaugluftfilter (7) frei bleibenden Abschnitt ein Ausblasraum (11) angeordnet ist, in den das Ventilatorrad (6) im Betrieb die Luft auf dem Weg hin zum Luftausblasbereich (4) des Gehäuses (2) einbläst.

2. Luftreinigungsvorrichtung (1) nach Anspruch 1, **dadurch gekennzeichnet, dass** der Lufteinsaugbereich (3) und/oder der Luftausblasbereich (4), von der Ventilatorrotationsachse (5) aus gesehen, am radial äußeren Rand (17) des Gehäuses (2) angeordnet sind.

3. Luftreinigungsvorrichtung nach einem der Ansprüche 1 oder 2, **dadurch gekennzeichnet, dass** der Lufteinsaugbereich (3) und der Luftausblasbereich (4) im Bereich am Außenumfang (2) des Gehäuses (2) im Mittenbereich des Außenumfangs (2) angeordneten, umlaufenden Ringnut (40) angeordnet sind.

4. Luftreinigungsvorrichtung (1) nach einem der Ansprüche 1 bis 3, **dadurch gekennzeichnet, dass** das Gehäuse (2) im Lufteinsaugbereich (3) und im Luftausblasbereich (4) eine Abfolge von Lamellen (18, 19) aufweist, wobei zwischen den Lamellen (18) im Lufteinsaugbereich (3) Luft in das Gehäuse (2) einsaugbar ist und zwischen den Lamellen (19) im Luftausblasbereich (4) Luft aus dem Gehäuse (2) ausblasbar ist.

5. Luftreinigungsvorrichtung (1) nach einem der Ansprüche 1 bis 4, **dadurch gekennzeichnet, dass,** in einer Draufsicht aus Richtung (8) parallel zur Ventilatorrotationsachse (5) gesehen, der oder die Einsaugluftfilter (7) den Innenraum (10), in dem das Ventilatorrad (6) angeordnet ist, nur unvollständig umgibt und C-förmig ausgebildet ist.

6. Luftreinigungsvorrichtung (1) nach einem der Ansprüche 1 bis 5, **dadurch gekennzeichnet, dass** im Ausblasraum (11) ein Ausblasluftfilter (12) angeordnet ist, der gegebenenfalls mit einer Vorrichtung zur Beduftung verbunden ist.

7. Luftreinigungsvorrichtung (1) nach einem der Ansprüche 1 bis 6, **dadurch gekennzeichnet, dass** ein Luftstrom (24) der vom Ventilatorrad (6) durch den Lufteinsaugbereich (3) in das Gehäuse (2) eingesaugten Luft bezüglich der Ventilatorrotationsachse (5) in radialer Richtung (9) in den oder die Einsaugluftfilter (7) hinein und anschließend zumindest auch in Richtungen (8) parallel zur Ventilatorrotationsachse (5) um eine Barrierewand (14) herum hin zum Ventilatorrad (6) verläuft.

8. Luftreinigungsvorrichtung (1) nach Anspruch 7, **dadurch gekennzeichnet, dass** die Barrierewand (14) einen Aufnahmeraum (16) für das Ventilatorrad (6) teilweise umschließt und das Ventilatorrad (6) in dem Aufnahmeraum (16) exzentrisch angeordnet ist.

9. Luftreinigungsvorrichtung (1) nach einem der Ansprüche 1 bis 8, **dadurch gekennzeichnet, dass** das Gehäuse (2, 2a, 2b, 2c) wahlweise als Tischgerät, als Wandgerät oder als Bodengerät ausgebildet ist.

10. Luftreinigungsvorrichtung (1) nach einem der Ansprüche 1 bis 9, **dadurch gekennzeichnet, dass** die Luftreinigungsvorrichtung (1) als Raumluftreiniger und/oder als Tischgerät ausgebildet ist, bei dem die Anordnung des Ventilators (6, 21, 23) ganz oder teilweise innerhalb eines oder mehrerer umgebender Filter (7) erfolgt.

## Claims

1. Air purification device (1) for personalised air supply having a housing (2) which has at least one air intake region (3) for drawing air into the housing (2) and at least one air blow-out region (4) for blowing air out of the housing (2), wherein at least one fan wheel (6) rotatable about a fan rotation axis (5) and at least one intake air filter (7) for filtering the air drawn in through the air intake region (3) are arranged in the housing (2), wherein a purified blow-out air stream (32) is blown out freely from the housing of the air purification device and can be directed individually against the body, and in particular against the face, of a user, wherein the housing (2) of the air purification device (1) has a disc-like design, and the air intake region (3) and the air blow-out region (4) are arranged in the same radial plane (39) on the circumference of the housing (2), **characterised in that** the housing (2) of the air purification device (1) has a circular or oval or polygonal outer contour in a view from direction (8) parallel to the fan rotation axis (5), **in that** the fan wheel (6) draws the air into the fan wheel (6) during operation of the air purification device (1) at least in a direction (8) parallel to the fan rotation axis (5) and blows the purified blow-out air stream (32) away from the fan wheel (6) in at least one direction (37) radially to the fan rotation axis (5), **in that** the fan rotation axis (5) is arranged perpendicularly to the direction (9) of the radial plane of the housing (2), and **in that** the fan wheel (6) is arranged in an interior (10) only partly surrounded by the intake air filter (7) or several intake air filters, and seen in the plan view from direction (8) parallel to the fan rotation axis (5), a blow-out space (11), in which the fan wheel (6) blows in the air on the path towards the air blow-out region (4) of the housing (2) during operation, is arranged in a section remaining free of the intake air filter (7).

2. Air purification device (1) according to claim 1, **characterised in that** the air intake region (3) and/or the air blow-out region (4), seen from the fan rotation axis (5), are arranged on the radially outer edge (17) of the housing (2).

3. Air purification device according to one of claims 1 or 2, **characterised in that** the air intake region (3) and the air blow-out region (4) are arranged in the region of a circumferential annular groove (40) arranged on the outer circumference (2) of the housing (2) in the central region of the outer circumference (2).

4. Air purification device (1) according to one of claims 1 to 3, **characterised in that** the housing (2) in the air intake region (3) and in the air blow-out region (4) has a sequence of lamellae (18, 19), wherein air can be drawn into the housing (2) between the lamellae (18) in the air intake region (3) and air can be blown out of the housing (2) between the lamellae (19) in the air blow-out region (4).

5. Air purification device (1) according to one of claims 1 to 4, **characterised in that** seen in a plan view from direction (8) parallel to the fan rotation axis (5), the intake air filter or filters (7) only incompletely surrounds the interior (10), in which the fan wheel (6) is arranged, and is designed to be C-shaped.

6. Air purification device (1) according to one of claims 1 to 5, **characterised in that** a blow-out air filter (12), which is optionally connected to a device for perfuming, is arranged in the blow-out space (11).

7. Air purification device (1) according to one of claims 1 to 6, **characterised in that** an air stream (24) of the air drawn into the housing (2) by the fan wheel (6) through the air intake region (3) runs with respect to the fan rotation axis (5) in radial direction (9) into the intake air filter or filters (7) and then at least also in directions (8) parallel to the fan rotation axis (5) around a barrier wall (14) towards the fan wheel (6).

8. Air purification device (1) according to claim 7, **characterised in that** the barrier wall (14) partly encloses a receiving space (16) for the fan wheel (6) and the fan wheel (6) is arranged eccentrically in the receiving space (16).

9. Air purification device (1) according to one of claims 1 to 8, **characterised in that** the housing (2, 2a, 2b, 2c) is designed alternatively as a table top unit, as a wall unit or as a floor unit.

10. Air purification device (1) according to one of claims 1 to 9, **characterised in that** the air purification device (1) is designed as a room air purifier and/or as a table top unit in which the arrangement of the fan (6, 21, 23) is effected wholly or partly within one or more surrounding filters (7).

## Revendications

1. Dispositif de purification d'air (1), pour l'apport d'air personnalisé comprenant un boîtier (2) qui comporte au moins une zone d'aspiration d'air (3) pour aspirer de l'air dans le boîtier (2) et une zone de soufflage d'air (4) pour souffler l'air hors du boîtier (2), au moins une roue de ventilateur (6) rotative autour d'un axe de rotation de ventilateur (5) et au moins un filtre d'air d'aspiration (7) pour filtrer l'air aspiré à travers la zone d'aspiration d'air (3) étant prévus dans le boîtier (2), un flux d'air de soufflage purifié (32) étant soufflé librement hors du boîtier du dispositif de purification d'air et pouvant être dirigé individuellement contre le corps d'un utilisateur, et en particulier contre le visage, le boîtier (2) du dispositif de purification d'air (1) présentant une configuration en forme de disque et la zone d'aspiration d'air (3) et la zone de soufflage d'air (4) étant disposées dans le même plan radial (39) sur le pourtour du boîtier (2), **caractérisé en ce que**
le boîtier (2) du dispositif de purification d'air (1) vu d'une direction (8) parallèle à l'axe de rotation de ventilateur (5) présente un contour extérieur circulaire ou ovale ou polygonal,
lors du fonctionnement du dispositif de purification d'air (1), la roue de ventilateur (6) aspire l'air au moins dans une direction (8) parallèle à l'axe de rotation de ventilateur (5) dans la roue de ventilateur (6) et souffle en éloignant de la roue de ventilateur (6) le flux d'air de soufflage purifié (32) dans au moins une direction (37) radiale par rapport à l'axe de rotation de ventilateur (5),
l'axe de rotation de ventilateur (5) est disposé perpendiculairement à la direction (9) du plan radial du boîtier (2), et
la roue de ventilateur (6) est disposée dans un espace intérieur (10) entouré en partie seulement par le filtre d'air d'aspiration (7) ou plusieurs filtres d'air d'aspiration, et, vu de la direction (8) parallèle à l'axe de rotation de ventilateur (5), un espace de soufflage (11) est disposé dans une section qui reste dégagée du filtre d'air d'aspiration (7), espace dans lequel en fonctionnement la roue de ventilateur (6) souffle en injectant l'air sur le chemin de la zone de soufflage d'air (4) du boîtier (2).

2. Dispositif de purification d'air (1) selon la revendication 1, **caractérisé en ce que** la zone d'aspiration d'air (3) et/ou la zone de soufflage d'air (4), vues de l'axe de rotation de ventilateur (5), sont disposées au bord radialement extérieur (17) du boîtier (2).

3. Dispositif de purification d'air selon l'une des revendications 1 ou 2, **caractérisé en ce que** la zone d'aspiration d'air (3) et la zone de soufflage d'air (4) sont disposées dans la zone d'une rainure annulaire périphérique (40) disposée dans la zone centrale de du pourtour extérieur du boîtier (2).

4. Dispositif de purification d'air selon l'une des revendications 1 à 3, **caractérisé en ce que** le boîtier (2) dans la zone d'aspiration d'air (3) et dans la zone de soufflage d'air (4) comporte une succession de lamelles (18, 19), de l'air pouvant être aspiré dans le boîtier (2) entre les lamelles (18) dans la zone d'aspiration d'air (3) et de l'air pouvant être soufflé hors du boîtier (2) entre les lamelles (19) dans la zone de soufflage d'air (4).

5. Dispositif de purification d'air (1) selon l'une des revendications 1 à 4, **caractérisé en ce que** vus dans la direction (8) parallèle à l'axe de rotation de ventilateur (5), le ou les filtres d'air d'aspiration (7) n'entourent que de manière incomplète l'espace intérieur (10) dans lequel est disposée la roue de ventilateur (6) et sont en forme de C.

6. Dispositif de purification d'air (1) selon l'une des revendications 1 à 5, **caractérisé en ce que** dans l'espace de soufflage (11) est disposé un filtre d'air de soufflage (12) qui est éventuellement relié à un dispositif de diffusion de parfum.

7. Dispositif de purification d'air (1) selon l'une des revendications 1 à 6, **caractérisé en ce qu'**un flux d'air (24) de l'air aspiré dans le boîtier (2) par la roue de ventilateur (6) à travers la zone d'aspiration d'air (3) s'écoule dans le ou les filtres d'air d'aspiration (7) dans la direction radiale (9) par rapport à l'axe de rotation de ventilateur (5) puis vers la roue de ventilateur (6) en passant autour d'une paroi barrière (14) au moins également dans des directions (8) parallèles à l'axe de rotation de ventilateur (5).

8. Dispositif de'purification d'air (1) selon la revendication 7, **caractérisé en ce que** la paroi barrière (14) entoure partiellement un logement (16) pour la roue de ventilateur (6) et la roue de ventilateur (6) est disposée de manière excentrée dans le logement (16).

9. Dispositif de purification d'air (1) selon l'une des revendications 1 à 8, **caractérisé en ce que** le boîtier (2, 2a, 2b, 2c) est conformé au choix en un appareil de table, un appareil au mur ou un appareil au sol.

10. Dispositif de purification d'air (1) selon l'une des revendications 1 à 9, **caractérisé en ce que** le dispositif de purification d'air (1) est conformé en un purificateur d'air ambiant et/ou un appareil de table, dans lequel l'agencement du ventilateur (6, 21, 23) se fait entièrement ou partiellement à l'intérieur d'un ou plusieurs filtres (7) environnants.
